# EUROPÄISCHE PATENTSCHRIFT

(11) **EP 2 212 673 B1**
(45) Veröffentlichungstag und Bekanntmachung des Hinweises auf die Patenterteilung: **13.06.2012**
(21) Anmeldenummer: 08853611.5
(22) Anmeldetag: 27.11.2008
(51) Int. Cl.: G01N 15/10, G01R 33/12, G01N 33/543

(54) **VORRICHTUNG ZUR MAGNETISCHEN DETEKTION VON EINZELPARTIKELN IN EINEM MIKROFLUIDISCHEN KANAL**
DEVICE FOR MAGNETIC DETECTION OF INDIVIDUAL PARTICLES IN A MICROFLUID CHANNEL
DISPOSITIF DE DÉTECTION MAGNÉTIQUE DE PARTICULES INDIVIDUELLES DANS UN CANAL MICROFLUIDIQUE

(30) Priorität: 30.11.2007 DE 102007057667
(43) Veröffentlichungstag der Anmeldung: 04.08.2010
(73) Patentinhaber: Siemens Aktiengesellschaft, 80333 München (DE)
(72) Erfinder: BÄR, Ludwig, 91056 Erlangen (DE); HAYDEN, Oliver, 91074 Herzogenaurach (DE)
(86) Internationale Anmeldenummer: PCT/EP2008/066305
(87) Internationale Veröffentlichungsnummer: WO 2009/068598

(56) Entgegenhaltungen:
- WO-A-01/27592
- WO-A-2007/092909
- US-A1- 2006 081 954
- JIANG Z ET AL: "An integrated microfluidic cell for detection, manipulation, and sorting of single micron-sized magnetic beads" JOURNAL OF APPLIED PHYSICS, AMERICAN INSTITUTE OF PHYSICS. NEW YORK, US, Bd. 99, Nr. 8, 26. April 2006 (2006-04-26), Seiten 8S105-1-08S105-3, XP012084910 ISSN: 0021-8979
- RIFE J C ET AL: "Design and performance of GMR sensors for the detection of magnetic microbeads in biosensors" SENSORS AND ACTUATORS A, ELSEVIER SEQUOIA S.A., LAUSANNE, CH, Bd. 107, Nr. 3, 1. November 2003 (2003-11-01), Seiten 209-218, XP004469965 ISSN: 0924-4247
- MITRELIAS ET AL: "Biological cell detection using ferromagnetic microbeads" JOURNAL OF MAGNETISM AND MAGNETIC MATERIALS, ELSEVIER SCIENCE PUBLISHERS, AMSTERDAM, NL, [Online] Bd. 310, Nr. 2, 16. März 2007 (2007-03-16), Seiten 2862-2864, XP022048775 EVL ISSN: 0304-8853
- LUDWIG F ET AL: "Magnetorelaxometry of magnetic nanoparticles with fluxgate magnetometers for the analysis of biological targets" JOURNAL OF MAGNETISM AND MAGNETIC MATERIALS, [Online] Bd. 293, 3. Mai 2005 (2005-05-03), Seiten 690-695, XP002520483 ISSN: 0304-8853 Gefunden im Internet: URL:http://www.sciencedirect.com/> [gefunden am 2009-03-19]

## Beschreibung

Die Erfindung betrifft eine Vorrichtung zur Detektion von Partikeln eines Fluids, insbesondere zur dynamischen Detektion der Partikel.

Mittels Immunofluoreszenz können mit einem FACS System (FACS: Fluorescence Activated Cell Sorting/Durchflusszytometrie) markierte Partikel, insbesondere auch Zellpopulationen analysiert und aufgetrennt werden (D. Huh et al., Physiol. Meas. 2005, 26, R73-R98). Dafür wird eine Zellpopulation durch eine Kapillare durchgepumpt, wo einzelne Zellen aufeinanderfolgend durch eine externe Optik auf ihre Fluoreszenzeigenschaften geprüft werden. Die Zellen werden unmittelbar nach der Fluoreszenzdetektion einzeln in Tröpfchen in einer Düse zerstäubt und die Tröpfchen mit markierten Zellen elektrische geladen. Durch eine Ablenkung der geladenen Tröpfchen in einem elektrischen Feld können die markierten Zellen aus einer großen Zellpopulation abgetrennt werden. FACS Geräte arbeiten hierbei mit mehrfachen Markern und mit Geschwindigkeiten von ~10⁵ Zellen/min. Diese Methode stellt den Industriestandard dar. Der Nachteil von FACS liegt in den hohen Anschaffungs- und Wartungskosten, sowie in der Komplexität des Systems, die geschultes Personal in der Bedienung erfordert.

Neben der FACS Auftrennung werden in der Literatur Systeme beschrieben die auf immunomagnetischen Prinzipien beruhen, wobei hier vorwiegend die Detektion markierter Zellen und weniger die Separation im Vordergrund stehen. Zum Einsatz kommen makroskopische Dipole und Quadrupol-Separatoren, sowie SQUIDS¹ (Superconducting Quantum Interference Device). Der Nachteil dieser magnetischen Ansätze sind die fehlende Praktikabilität (keine Miniaturisierung möglich) und die hohen Kosten. Dipol- und Quadrupolsysteme sind analog zu FACS aufwendige und teure Instrumente, die aufgrund Ihrer Größe nur kleine Wiederfindungsraten von markierten Zellen aufweisen. SQUIDs müssen auf unter 100 K gekühlt werden, um funktionstüchtig zu sein. Zudem ist durch die Kühlung ein komplexer Aufbau des Detektors notwendig, der die hohe Sensitivität von SQUIDs konterkariert.

In modernen Zellseparationsystemen wird MACS (Magnetic Activated Cell Sorting) verwendet. Hier werden paramagnetische Nanopartikel, die mit monoklonalen Antikörpern beschichtet sind, mit einer Zellsuspension vermischt. Die Antikörper binden an das spezifische Antigen auf der Zelloberfläche. Durchläuft die Zellsuspension ein starkes magnetisches Feld in einer Säule, bleiben die Zell-Nanopartikel-Komplexe in der Säule, während die freien Zellen augenblicklich hindurch fließen (negative Selektion). Wird die Säule von dem magnetischen Feld fortgenommen, können die Zell-Nanopartikel-Komplexe zurückgewonnen werden (positive Selektion). Zellen die an Kügelchen haften sind lebensfähig und der Kügelchen-Antikörper-Komplex kann von der Zelloberfläche entfernt werden. MACS ist vollständig FACS-kompatibel. Nach der magnetischen Separation (100-fache Anreicherung von bis zu 10⁹ Zellen in 15 min) kann gleich die durchflusszytometrische Analyse mit Fluoreszenzmarkierung erfolgen. Der Nachteil dieses Prozesses liegt in der Notwendigkeit einer doppelten Markierung (magnetisch und Fluoreszenz), um eine kostengünstige Analyse/verfeinerte Separation der Zellen durchzuführen.

Das Dokument US2006/081954 A1 beschreibt ein System zur dynamischen Detektion und Selektion superparamagnetisch gekennzeichneter Zellen, einen Mikrofluidikkanal und eine um diesen angeordnete Wheatstone'sche Brückenschaltung mit magnetoresistiven Elementen, wobei der Mikrofluidikkanal in der Brückenschaltung so angeordnet ist, dass die durch den Mikrofluidikkanal fließenden magnetisch gekennzeichneten Zellen den Abgleich der Brückenschaltung messbar beeinflussen. Ferner sind zwei Brückenelemente außerhalb des Detektionsbereiches des Mikrofluidikkanals sich gegenüberliegende Diagonalelemente der Wheatstone-Geometrie und haben die Elemente der Brücke einen geometrischen Abstand von mindestens der Zellgröße.

Aufgabe der vorliegenden Erfindung ist es deshalb, die Nachteile des Standes der Technik zu überwinden, eine kostengünstige und genaue Vorrichtung und ein Verfahren zur Detektion magnetisch gekennzeichneter Partikel, insbesondere von Zellen, zu schaffen.

Lösung der Aufgabe und Gegenstand der Erfindung ist daher eine Vorrichtung zur dynamischen Detektion und Selektion superparamagnetisch gekennzeichneter Zellen gemäß dem Anspruch 1, einen Mikrofluidikkanal und eine um diesen angeordnete Wheatstone'sche Brückenschaltung mit zumindest einem magnetoresistiven Element umfassend, wobei der Mikrofluidikkanal in der Brückenschaltung so angeordnet ist, dass die durch den Mikrofluidikkanal fließenden magnetisch gekennzeichneten Partikel den Abgleich der Brückenschaltung messbar beeinflussen.

Der magnetoresistive Sensor wird also durch die Anordnung von zumindest einem magnetoresistiven Element in der Form einer Wheatstone'schen Messbrücke realisiert. Zum dynamischen Nachweis von magnetischen Partikeln sind die Elemente der Brücke in einem geometrischen Abstand von mindestens der Partikel- bzw. Zellgröße.

Dabei sind zwei Diagonalelemente innerhalb und zwei Diagonalelemente außerhalb des Detektionsbereiches des Mikrofluidikkanals angeordnet, wodurch sich die Ortsauflösung verdoppelt, oder es sind 3 Brückenelemente außerhalb des Mikrofluidikkanals und ein Brückenelement innerhalb des Detektionsbereiches des Mikrofluidikkanals angeordnet, wodurch sich die Ortsauflösung vervierfacht.

Außerdem ist Gegenstand der Erfindung ein Verfahren zur dynamischen Detektion und Selektion superparamagnetisch gekennzeichneter Zellen gemäß dem Anspruch 3, wobei durch einen Mikrofluidikkanal magnetisch gekennzeichnete Partikel fließen, die durch das von ihnen erzeugte magnetische Streufeld zumindest einen magnetoresistiven Widerstand der um den Mikrofluidikkanal angeordneten Wheatstone'schen Brückenschaltung derart beeinflussen, dass eine messbare Auslenkung der Brücke resultiert.

Die vorgeschlagene Lösung basiert auf dem Einsatz von magnetoresistiven Bauelementen, die in Mikrofluidikkanälen integriert werden. Die Zelldetektion erfolgt - anders als bei allen immunomagnetischen Ansätzen bisher - dynamisch während des Flusses von Zellen durch einen Mikrofluidikkanal.

Dabei werden in dem Verfahren wertvolle zusätzliche Informationen über die superparamagnetisch gekennzeichneten Zellen oder Zelltypen durch Bestimmung der unterschiedlichen Relaxationszeiten der Magnetisierung durch zeitlich aufgelöste Messungen mit dem magnetoresistiven Sensor gewonnen, und die Vorrichtung umfasst geeignete Mittel zur Gewinnung dieser Informmationen.

Vorteilhafterweise werden zumindest zwei Arten magnetisch gekennzeichneter Partikel eingesetzt, zum einen permanent magnetisierte Partikel, deren Dipolmoment auch nach Wegfall eines zur Magnetisierung nötigen äußeren Magnetfeldes bestehen bleibt und zum anderen temporär magnetisch gekennzeichnete Partikel, sogenannte superparamagnetische Partikel, insbesondere auch Nanopartikel. Durch ein der Vorrichtung vor geschaltetes äußeres Magnetfeld werden diese Partikel temporär magnetisiert, und die Magnetisierung klingt in Abhängigkeit des superparamagnetischen Partikel mit einer charakteristischen Abklingzeit ab. Der Unterschied in der Abklingzeit ermöglicht, die hier beschriebene dynamische Detektion und Selektion vorzunehmen.

Letztgenannte superparamagnetisch markierte Partikel werden bei der hier beschriebenen dynamischen Detektion und Selektion eingesetzt.

Ferromagnetische Partikel sind demgegenüber nicht bevorzugt, es besteht die Gefahr, dass sie klumpen und in diesem Ansatz den Sensor stören bzw. den Mikrofluidikkanal blockieren. Streufeld heißt, dass das durch die magnetisch gekennzeichneten Partikel erzeugte Feld durch ein externes Feld induziert worden ist.

Superparamagnetische Partikel werden dadurch erzeugt, dass sie im externen Feld magnetisch polarisiert werden. Wenn das externe Feld weg ist bleibt mit einer gewissen Abklingzeit kein Dipol übrig.

Die dynamische Messung erfolgt mittels eines Mikrofluidikkanals. Der Mikrofluidikkanal ist an die Größe der untersuchten Zellen angepasst, um eine Einzeldetektion mit hoher Wiederfindungsrate zu ermöglichen. Nach einer bevorzugten Ausführungsform ist der Mikrofluidikkanal mit mikromechanischen Mitteln abgeformt, beispielsweise durch Ätzen einer Silizium Scheibe. Die Anpassung des Mikrofluidikkanals erfolgt so, dass jedes Partikel oder jede Zelle einzeln durch den Mikrofluidikkanal fließt, also nicht mehrere Partikel nebeneinander Platz in dem Kanal haben. Beispielsweise kann der Mikrofluidikkanal auch über eine Abformtechnik hergestellt werden, so dass er beispielsweise durch Gießen eines Silikons über einen Siliziumstempel hergestellt wird. Bevorzugt ist der Mikrofluidikkanal aus transparentem Material.

Zusätzlich zu der dynamischen Detektion und Selektion kann auch eine weitere optische, elektrische, magnetische und/oder andere Detektionsmethode eingesetzt werden, um das im Mikrokanal befindliche Fluid zu analysieren.

Die Detektionsmöglichkeiten:
a) Optisch:
   Durch Mikroskopie, Absorptions- oder Fluoreszenzmessung werden alle Zellen in einem Mikrofluidikkanal detektiert und der Anteil markierter Zellen durch Vergleichsmessung mit einem magnetoresistiven Bauelement berechnet.
b) Elektrisch:
   Durch Impedanzspektroskopie, kapazitive, resistive oder dielektrophoretische Messmethoden wird analog zur optischen Bestimmung das Durchströmen einzelner Zellen im Mikrofluidikkanal gezählt und wiederum der Anteil magnetisch markierter Zellen durch Differenzmessung mit registrierten Zahl an markierten Zellen durch Messung mit einem magnetoresistivem Detektor berechnet.
c) Magnetisch (1 Typ von magnetischen Nanopartikel):
   Jede Zelle, die durch den - auf die Zelldimension angepassten - Mikrofluidikkanal strömt, wird im Bereich des magnetoresistivem Sensors aufgrund des Zellvolumens die wässrige Lösung verdrängt und damit weniger ungebundene magnetische Nanopartikel vorhanden sein. Diese temporäre Verarmung an Nanopartikel führt zu einem verringerten Messsignal und erlaubt die Detektion unmarkierter Zellen. Im Falle einer markierten Zelle wird ein großes Messsignal detektiert werden, da die magnetischen Nanopartikel an der Zelloberfläche oder im Zellinneren angereichert sind.
d) Magnetorelaxometrie:
   Das Abklingverhalten des Magnetischen Moments von Nanopartikeln wird bestimmt durch die Größe des Partikels selbst und durch die Bindung an andere Teilchen oder Substrate. Durch Bestimmung der unterschiedlichen Relaxationszeiten durch zeitlich aufgelöste Messungen mit dem magnetoresistivem Sensor können wertvolle zusätzliche Informationen gewonnen werden. Z.B. können gebundene von ungebundenen Zellen unterschieden werden. Es ist denkbar, verschiedene Zelltypen oder Zellen mit unterschiedlichen Antigenbindungen auszurüsten und jeweils Nanopartikel mit unterschiedlicher Größensortierung anzubinden. Auch verschieden große Zellen als Liganden an der gleichen Sorte von magnetischen Nanopartikel lassen sich so unterschieden.

Der schematische Aufbau ist in den Figuren gezeigt. Eine Wheatstone'sche Brückenanordnung von in der Regel 4 magnetoresistiven Widerständen wird so angeordnet, dass zwei Widerstände unterhalb eines Mikrofluidikkanals geführt werden und zwei Widerstände außerhalb des Mikrofluidikbereichs verbleiben, um eine maximale Verstimmung der Brücke durch magnetmarkierte Zellen zu erreichen. Die Anordnung der 4 Elemente ist jedoch veränderbar und je nach Anordnung kann die Signalempfindlichkeit oder die Ortsauflösung der Vorrichtung und des Verfahrens optimiert werden.

Zudem ist es nicht zwingend, dass 4 magnetoresistive Elemente in der Brückenschaltung realisiert werden. Ebenso gut können auch 2 magnetoresistive Elemente mit zwei herkömmlichen Widerständen in der Brücke kombiniert vorliegen. Oder eine beliebige andere Kombination, solange zumindest ein magnetoresistives Element in der Brückenschaltung vorhanden ist.

Die magnetoresistiven Elemente können sowohl herkömmliche magnetoresistive Elemente sein, wie sie beispielsweise in der DE 102 02 287 beschrieben werden, es kann sich aber auch um organische magnetoresistive Elemente handeln, wie beispielsweise aus der DE 10 2006 019 482 bekannt.

Mittels magnetoresistiver Messungen ist eine Analyse und Separation von Partikel, beispielsweise heterogener Zellpopulationen auf der Basis zellulärer Merkmale zugänglich. Für die Auftrennung dieser heterogenen Zellpopulationen werden immunomagnetische Marker (Nanopartikel gebunden an einen Rezeptor oder Ligand) herangezogen. Die markierten Zellen werden in einem externen Magnetfeld magnetisiert und erzeugen dadurch ein zusätzliches magnetisches Streufeld, welches durch ein magnetoresistives Bauteil detektiert werden kann. Durch den Einsatz von Magnetorelaxometrie (zeitl. Abklingverhalten des Streufeldes) lassen sich ungebundene und unterschiedliche gebundene magnetischen Nanopartikeln voneinander unterscheiden. Weitere Fluoreszenzmarkierungen oder ähnliches sind für die Auftrennung bzw. Analyse der Zellpopulation nicht notwendig.

Die markierten Zellen werden durch ein magnetoresistives Bauelement detektiert, wohingegen die unmarkierten Zellen optisch, elektrisch oder magnetoresistiv erfasst werden können.

Die Vorrichtung und das Verfahren nach der Erfindung haben das Potential bei der konventionellen FACS Analyse die Fluoreszenzmessung zu ersetzen. Bei dieser eingangs erwähnten Art der Zelldetektion mit Fluoreszenz werden die Zellen in eine kleine Kapillare eingeleitet. Die Fluoreszenzdetektion erfolgt diskret an einzelnen Zellen. Die einzelnen Zellen werden in kleinen Tröpfchen zerstäubt und abhängig von einer Fluoreszenzdetektion elektrisch geladen. Die geladenen Tröpfchen werden in einem Kollektor abgelenkt und in einem Gefäß gesammelt.

Der GMR Sensor wird hierbei vor dem Zerstäuber eingesetzt. Messungen bis in den MHz Bereich können mit diesem Aufbau erfolgen. Der Vorteil dieses Aufbaus liegt in der massiven Miniaturisierbarkeit und Parallelisierungsmöglichkeit. Neben der Separation mit Hilfe eines Zerstäubers kann zudem eine Analyse der Zellpopulation durchgeführt werden.

In bisher bekannten Anwendungen der GMR-Sensorik in Biologie und Medizin (DNA-Sensor) wird immobilisiertes, magnetisch markiertes Material detektiert. Man verwendet dabei ein statisches Ausleseverfahren. Für die in dieser Erfindung offenbarte dynamische Messung von Einzelanalyten ist eine hochfrequente Abfrage des Sensorstatus vorteilhaft. Dies wird z. B. bewerkstelligt durch eine Feldabtastung des Sensors im relevanten Bereich der Charakteristik bei Frequenzen typischer Weise wesentlich größer 1 kHz. Eine Abweichung in der Sensoramplitude oder ein Sprung in der Sensorempfindlichkeit (differenziertes Signal) sind z. B. Kennzeichen für eine Ereignis-Detektion.

Dieses Ausleseverfahren ermöglicht darüber hinaus den Einsatz von elektronischer Lock-in-Filterung für die Aufbereitung des Nutzsignals. Dadurch kann die Empfindlichkeit des Ereignis-Nachweises deutlich erhöht werden.

Zum dynamischen Nachweis von magnetischen Partikeln sind die Elemente der Brücke also in einem geometrischen Abstand von mindestens der Partikelgröße. Die Brücke hat dann eine effektive Ausdehnung von vier Partikeldurchmessern. Die Detektion von eng aufeinanderfolgenden Partikeln wird dadurch zum Problem. Unter der Voraussetzung, dass der Fluidikkanal mikrotechnologisch präzise hergestellt und auf einem Trägerchip mit hoher Genauigkeit zu positioniert werden kann, lässt sich die Geometrie der Brückenschaltung so modifizieren, dass jeweils zwei Diagonalelemente der Wheatstone-Geometrie innerhalb und die beiden anderen Diagonalelemente außerhalb der Mikrofluidikzelle zu liegen kommen. Dadurch wird die Ortauflösung für die Partikeldetektion verdoppelt.

In einem anderen Ausführungsbeispiel werden drei Brückenelemente außerhalb des Detektionsbereiches platziert. Dadurch halbiert sich zwar die Signalempfindlichkeit des Sensors. Die Ortauflösung dagegen vervierfacht sich.

Im Folgenden wird die Erfindung noch anhand von Figuren näher erläutert:
- Figur 1: zeigt das Schaltbild einer Ausführungsform der Erfindung,
- Figur 2: zeigt die magnetische Relaxation von magnetischen Nanopartikeln
- Figuren 3 und 4: zeigen ein Beispiel für eine hochfrequente Abfrage des Sensorstatus der Messung,
- Figur 5: ein Diagramm der Sensorempfindlichkeit oder Sensitivität.

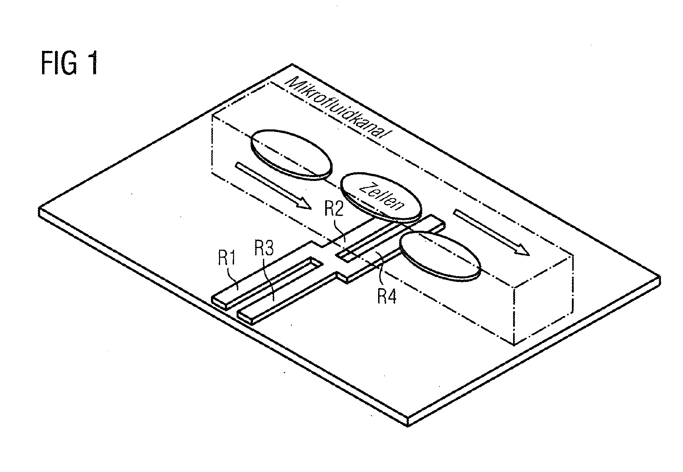

In Figur 1 erkennt man einen beispielhaften Aufbau für eine geometrische Anordnung der Brücke, bei der 2 Brückenelemente also beispielsweise zwei magnetoresistive Widerstände R1 und R3 außerhalb des Mikrofluidikkanals 1 und 2 Brückenelemente, wiederum entweder elektrische oder magnetoresistive Widerstände R2 und R4 unterhalb des Mikrofluidikkanals also innerhalb des so genannten Detektionsbereiches des Mikrofluidikkanals angeordnet sind. Zumindest ein Widerstand ist ein magnetoresistiver Widerstand. Im Fluidikkanal 1 sind einzelne Zellen 2 zu erkennen. Die Pfeile indizieren die Richtung, in die das Fluid fließt.

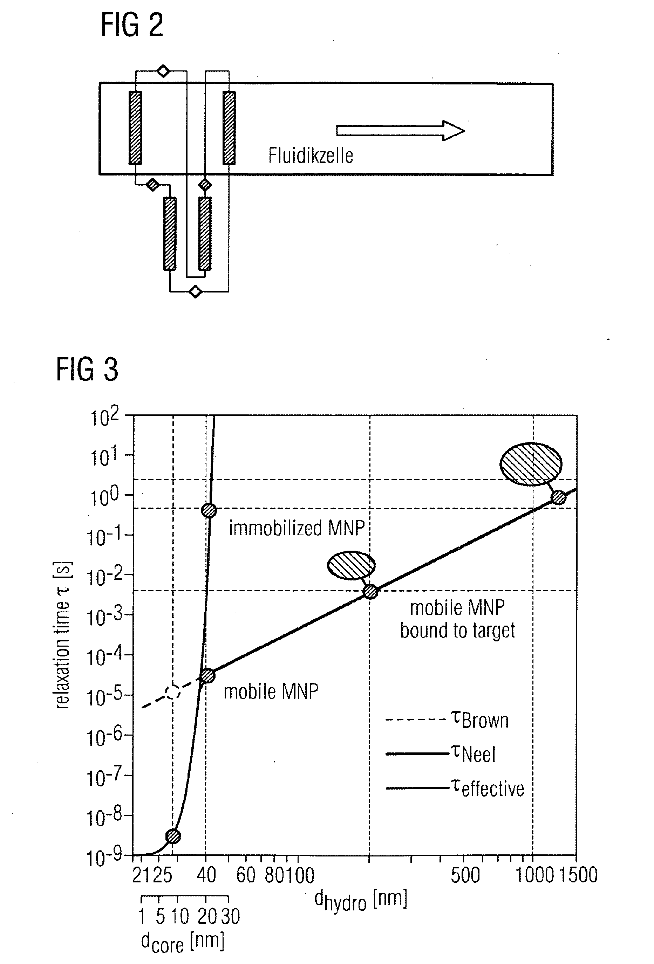

Figur 2 zeigt eine abgewandelte Anordnung der Wheatstonebrücke. Zu erkennen ist wieder der Fluidikkanal 1 und wieder 4 Widerstände, R1 bis R4, die in einer Wheatstone'schen Brückenschaltung angeordnet sind.

In Figur 3 ist beispielhaft gezeigt, wie die magnetische Relaxation von magnetischen Nanopartikeln gemessen wird. Diese Figur zeigt die Relaxationszeit als Funktion des(hydrodynamischen) Partikeldurchmessers, wobei letzterer z.B. in einem Ausschnitt auf Seite 693, rechte Spalte, Zeilen 13-18, aus der Veröffentlichung von F. Ludwig, E. Heim, S. Mauselein, D. Eberbeck and M. Schilling:"Magnetorelaxometry of magnetic nanoparticles with fluxgate magnetometers for the analysis of biological targets" J Magnet Magn. Mat. 2005;293(1):690-695 erwähnt ist.

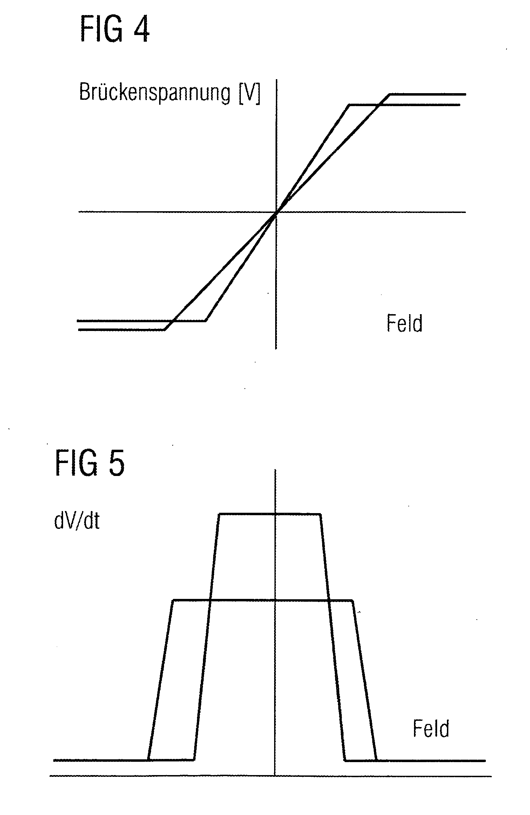

In Figur 4 zeigt ein Diagramm der Signalamplitude und Figur 5 ein Diagramm der Sensorempfindlichkeit oder Sensitivität.

Fluorophore werden können bei Langzeituntersuchungen verstoffwechselt werden oder bleichen aus. Magnetische Nanopartikel sind bio/chemisch resistent und optisch indifferent.

Damit kann zum Beispiel in einem miniaturisierten System kontinuierlich die Reaktionskinetik verfolgt werden.

Die Vorrichtung und das Verfahren nach der Erfindung eignen sich unter anderem für Langzeitstudien. Die chemisch stabilen Nanopartikel können über lange Zeiträume einem Zellexperiment zUJgesetzt werden.

Beispielsweise kann damit die Exprimierung von Oberflächenproteinen kontinuierlich studiert werden. Weiter Anwendungen können sein Zelladhäsionstests, Toxizitätstests und mehr.

Mit der Erfindung wird erstmals ein dynamisches und gut miniaturisierbares Verfahren zur Detektion und Selektion magnetisierter Zellen gezeigt.

## Patentansprüche

1. Vorrichtung zur dynamischen Einzel detektion und selektion superparamagnetisch gekennzeichneter Zellen, einen an die Größe der untersuchten Zellen angepassten Mikrofluidikkanal und eine um diesen angeordnete Wheatstone'sche Brückenschaltung mit zumindest einem magnetoresistiven Element umfassend, wobei der Mikrofluidikkanal in der Brückenschaltung so angeordnet ist, dass die durch den Mikrofluidikkanal fließenden magnetisch gekennzeichneten Zellen den Abgleich der Brückenschaltung messbar beeinflussen, wobei 3 Brückenelemente außerhalb des Mikrofluidikkanals und ein Brückenelement innerhalb des Detektionsbereiches des Mikrofluidikkanals angeordnet sind oder zwei Brückenelemente innerhalb und zwei Brückenelemente außerhalb des Detektionsbereiches des Mikrofluidikkanals angeordnet sind, wobei zwei Brückenelemente außerhalb des Detektionsbereiches des Mikrofluidikkanals sich gegenüberliegende Diagonalelemente der Wheatstone-Geometrie sind, und wobei die Elemente der Brücke in einem geometrischen Abstand von mindestens der Zellgröße sind, und wobei diese Vorrichtung ferner **dadurch gekennzeichnet ist, dass** sie Mittel zur Gewinnung wertvoller zusätzlicher Informationen über die superparamagnetisch gekennzeichneten Zellen oder Zelltypen durch Bestimmung der unterschiedlichen Relaxationszeiten der Magnetisierung durch zeitlich aufgelöste Messungen mit dem magnetoresistiven Sensor umfasst.

2. Vorrichtung nach Anspruch 1, wobei der Mikrofluidikkanal an die Größe der Partikel so angepasst ist, dass die Partikel den Kanal einzeln passieren.

3. Verfahren zur dynamischen Einzel detektion und Selektion superparamagnetisch gekennzeichneter Zellen, wobei durch einen an die Größe der untersuchten Zellen angepassten Mikrofluidikkanal superparamagnetisch gekennzeichnete Zellen fließen, die durch das von ihnen erzeugte magnetische Streufeld zumindest einen magnetoresistiven Widerstand der um den Mikrofluidikkanal angeordneten Wheatstone'schen Brückenschaltung derart beeinflussen, dass eine messbare Auslenkung der Brücke resultiert, wobei die Zelldetektion dynamisch während des Flusses von Zellen durch einen Mikrofluidikkanal erfolgt und wobei die Zellen durch ein vor geschaltetes äußeres Magnetfeld temporär magnetisiert werden und die Magnetisierung in Abhängigkeit der superparamagnetisch gekennzeichneten Zellen mit einer charakteristischen Abklingzeit abklingt, und wobei dieses Verfahren **dadurch gekennzeichnet ist, daß** durch Bestimmung der unterschiedlichen Relaxationszeiten durch zeitlich aufgelöste Messungen mit dem magnetoresistiven Sensor wertvolle zusätzliche Informationen über die superparamagnetisch gekennzeichneten Zellen oder Zelltypen gewonnen werden.

4. Verfahren nach Anspruch 3, das mit einem weiteren Verfahren zur Detektion, wie einem optischen elektrischen oder magnetischen Verfahren zur Detektion und/oder Selektion gekoppelt ist.

5. Verfahren nach einem der Ansprüche 3 oder 4, das bei der konventionellen "Flourescence Activated Cell Sorting", FACS-Analyse eingesetzt wird.

6. Verwendung einer Vorrichtung nach einem der Ansprüche 1 oder 2 oder des Verfahrens nach einem der Ansprüche 3 bis 5 bei Zelladhäsionstests und/oder Toxizitätstests.

## Claims

1. Device for dynamic individual detection and selection of superparamagnetically labelled cells, comprising a microfluidic channel matched to the size of the examined cells and, disposed around same, a Wheatstone bridge circuit with at least one magnetoresistive element, said microfluidic channel being disposed in the bridge circuit such that the magnetically labelled cells flowing through the microfluidic channel measurably influence the balance of the bridge circuit, wherein 3 bridge elements are disposed outside the microfluidic channel and one bridge element is disposed inside the detection region of the microfluidic channel or two bridge elements are disposed inside and two bridge elements outside the detection region of the microfluidic channel, two bridge elements outside the detection region of the microfluidic channel being opposing diagonal elements of the Wheatstone geometry, and wherein the elements of the bridge are at a geometric distance of at least the cell size and wherein this device is further **characterised in that** it comprises means for obtaining valuable additional information about the superparamagnetically labelled cells or cell types by determining the different magnetisation relaxation times by time-resolved measurements with the magnetoresistive sensor.

2. Device according to claim 1, wherein the microfluidic channel is matched to the size of the particles such that the particles pass individually through the channel.

3. Method for dynamic individual detection and selection of superparamagnetically labelled cells, wherein superparamagnetically labelled cells flow through a microfluidic channel matched to the size of the examined cells, which, due to the magnetic stray field generated by them, influence at least one magnetoresistive resistor of the Wheatstone bridge circuit disposed around the microfluidic channel so as to produce a measurable deflection of said bridge, wherein cell detection takes place dynamically during the flow of cells through a microfluidic channel and wherein the cells are temporarily magnetized by an upstream external magnetic field and the magnetization decays with a characteristic decay time as a function of the superparamagnetically labelled cells and wherein said method is **characterised in that** valuable additional information about the superparamagnetically labelled cells or cell types is obtained by determining the different relaxation times by time-resolved measurements with the magnetoresistive sensor.

4. Method according to claim 3, which is coupled with another method for detection, such as an optical, electrical or magnetic method for detection and/or selection.

5. Method according to one of claims 3 or 4, which is used in conventional Fluorescence Activated Cell Sorting (FACS) analysis.

6. Use of a device according to one of claims 1 or 2 or of the method according to one of claims 3 to 5 for cell adhesion tests and/or toxicity tests.

## Revendications

1. Dispositif de détection individuelle et de sélection dynamique de cellules caractérisées superparamagnétiquement, comprenant un canal microfluidique adapté à la dimension des cellules étudiées et un circuit en pont de Wheatstone, qui est disposé autour de celui-ci et qui a au moins un élément magnétorésistif, le canal microfluidique étant disposé dans le circuit en pont, de manière à ce que les cellules caractérisées magnétiquement s'écoulant dans le canal microfluidique influent de manière mesurable sur l'équilibre du circuit en pont, dans lequel trois éléments du pont sont disposés à l'extérieur du canal microfluidique et un élément du pont est disposé à l'intérieur de la zone de détection du canal microfluidique ou deux éléments du pont sont disposés à l'intérieur et deux éléments du pont à l'extérieur de la zone de détection du canal microfluidique, deux éléments du pont à l'extérieur de la zone de détection du canal microfluidique sont des éléments opposés en diagonale de la géométrie de Wheatstone et les éléments du pont sont à une distance géométrique d'au moins la dimension de cellule, et dans lequel ce dispositif est **caractérisé, en outre, par le fait qu'**il comprend des moyens d'obtention d'informations supplémentaires précieuses sur les cellules caractérisées superparamagnétiquement ou sur des types de cellules, par détermination des temps de relaxation différents de l'aimantation par des mesures résolues dans le temps par le capteur magnétorésistif.

2. Dispositif suivant la revendication 1, dans lequel le canal microfluidique est adapté à la dimension des particules, de manière à ce que les particules passent individuellement dans le canal.

3. Procédé de détection individuelle et de sélection dynamique de cellules caractérisées superparamagnétiquement, dans lequel, dans un canal microfluidique adapté à la dimension des cellules étudiées, passent des cellules caractérisées superparamagnétiquement, qui influencent, par le champ de dispersion magnétique qu'elles produisent, au moins une résistance magnétorésistive du circuit en pont de Wheatstone disposé autour du canal microfluidique, de manière à provoquer une déviation mesurable du pont, la détection des cellules s'effectuant dynamiquement pendant le flux de cellules passant dans un canal microfluidique et les cellules étant aimantées temporairement par un champ magnétique extérieur monté en amont, et l'aimantation décroissant avec un temps de décroissance caractéristique en fonction des cellules caractérisées superparamagnétiquement, et dans lequel ce procédé est **caractérisé par le fait que**, par la détermination des temps de relaxation différents par des mesures résolues dans le temps par le capteur magnétorésistif, on obtient des informations supplémentaires précieuses sur les cellules caractérisées superparamagnétiquement ou sur les types de cellules.

4. Procédé suivant la revendication 3, qui, pour la détection et/ou la sélection, est couplé à un autre procédé de détection, comme un procédé optique, électrique ou magnétique.

5. Procédé suivant l'une des revendications 3 ou 4, qui est utilisé dans l'analyse FACS "Flourescence Activated Cell Sorting" classique.

6. Utilisation d'un dispositif suivant l'une des revendications 1 ou 2 ou du procédé suivant l'une des revendications 3 à 5, dans des essais d'adhérence de cellules ou dans des essais de toxicité.
